# EUROPEAN PATENT APPLICATION

(11) **EP 4 300 051 A2**
(43) Date of publication of application: **03.01.2024**
(21) Application number: 23176754.2
(22) Date of filing: 01.06.2023
(51) Int. Cl.: G01F 23/24, A61L 9/14, B05B 17/06, F24F 6/12, F24F 8/24

(54) **SPRAY DEVICE AND CONTROL METHOD**

(30) Priority: 30.06.2022 JP 2022105597
(71) Applicant: Funai Electric Co., Ltd., Daito, Osaka, 574-0013 (JP)
(72) Inventor: HAMAOKA, Mika, Daito, Osaka, 574-0013 (JP)
(74) Representative: Kurig, Thomas

(57) **Abstract**

A spray device (10) includes a holding part (20), a spray part (40), and a detection part (50). The holding part (20) is capable of holding a liquid (1) therein. The spray part (40) atomizes and sprays the liquid (1) held in the holding part (20). The detection part (50) detects a liquid level of the liquid held in the holding part (20). The detection part (50) includes a first electrode (51) and a second electrode (52) provided inside the holding part (20), and an application part (53). The application part (53) applies a first voltage between the first electrode (51) and the second electrode (52) with the first electrode as an anode and the second electrode as a cathode, and then applies a second voltage between the first electrode (51) and the second electrode (52) with the first electrode as the cathode and the second electrode as the anode.

## Description

### BACKGROUND

### [Technical Field]

The disclosure relates to a spray device and the like for spraying a liquid.

### [Related Art]

A spray device and the like for spraying a liquid are conventionally known. As an example of the spray device, Patent Document 1 discloses an air purifier in which a first electrode is energized as a positive electrode and a second electrode is energized as a negative electrode, and then the first electrode is energized as the negative electrode and the second electrode is energized as the positive electrode.

### [Related Art Document]

### [Patent Document]

[Patent Document 1] Japanese Patent Application Laid-Open No. 2012-075667

### SUMMARY

### [Problem to Be Solved]

However, in the air purifier of Patent Document 1, it is not assumed to detect a liquid level of the liquid using the first electrode and the second electrode, and there is a problem that the liquid level of the liquid cannot be detected.

The disclosure has been made to solve the above problem, and an objective thereof is to provide a spray device and the like capable of detecting a liquid level of a liquid while suppressing deterioration of electrodes.

### [Means for Solving Problem]

To achieve the above objective, a spray device according to an embodiment of the disclosure includes a holding part, a spray part, and a detection part. The holding part is capable of holding a liquid inside the holding part. The spray part atomizes and sprays the liquid held in the holding part. The detection part detects a liquid level of the liquid held in the holding part. The detection part includes a first electrode, a second electrode, and an application part. The first electrode and the second electrode are provided inside the holding part. The application part applies a first voltage between the first electrode and the second electrode with the first electrode as an anode and the second electrode as a cathode, and then applies a second voltage between the first electrode and the second electrode with the first electrode as the cathode and the second electrode as the anode.

According to this embodiment, with the detection part which detects the liquid level of the liquid held in the holding part, it is possible to detect the liquid level of the liquid. Further, since the detection part includes the application part which applies the first voltage between the first electrode and the second electrode with the first electrode as the anode and the second electrode as the cathode, and then applies the second voltage between the first electrode and the second electrode with the first electrode as the cathode and the second electrode as the anode, it is possible to suppress deterioration of the electrodes. In this manner, it is possible to detect the liquid level of the liquid while suppressing deterioration of the electrodes.

For example, in the spray device according to an embodiment of the disclosure, the application part may apply the second voltage such that a waveform of the second voltage is an inverted waveform of a waveform of the first voltage.

According to this embodiment, it is possible to detect the liquid level of the liquid while further suppressing deterioration of the electrodes.

For example, in the spray device according to an embodiment of the disclosure, the application part may intermittently perform an operation of applying the first voltage and then applying the second voltage.

According to this embodiment, it is possible to repeatedly detect the liquid level of the liquid while further suppressing deterioration of the electrodes.

For example, in the spray device according to an embodiment of the disclosure, the application part may apply the first voltage and the second voltage in succession.

According to this embodiment, it is possible to detect the liquid level of the liquid while further suppressing deterioration of the electrodes.

For example, in the spray device according to an embodiment of the disclosure, the application part may intermittently perform an operation of applying the first voltage and the second voltage in succession at a time interval longer than a time of applying the first voltage and the second voltage in succession.

According to this embodiment, it is possible to repeatedly detect the liquid level of the liquid while further suppressing deterioration of the electrodes.

For example, in the spray device according to an embodiment of the disclosure, the application part may not apply the first voltage and the second voltage in a case where the liquid is not being sprayed by the spray part.

According to this embodiment, it is possible to detect the liquid level of the liquid while further suppressing deterioration of the electrodes.

For example, in the spray device according to an embodiment of the disclosure, the spray part may include a vibrator which vibrates and atomizes the liquid, and an air blowing part which blows air to the liquid atomized by the vibrator.

According to this embodiment, it is possible to detect the liquid level of the liquid while suppressing deterioration of the electrodes, and it is possible to easily spray the liquid held in the holding part.

For example, the spray device according to an embodiment of the disclosure may further include a control part which controls the spray part. The control part may drive the air blowing part in conjunction with the vibrator.

According to this embodiment, since the air blowing part can be driven when the vibrator is vibrating, it is possible to efficiently spray the liquid held in the holding part.

For example, in the spray device according to an embodiment of the disclosure, the control part may stop air blowing of the air blowing part in a case where vibration of the vibrator has stopped.

According to this embodiment, it is possible to suppress air blowing of the air blowing part when the liquid is not atomized and air blowing is not required.

For example, in the spray device according to an embodiment of the disclosure, taking a spray amount of the liquid required to be sprayed as A, an atomization rate of the liquid due to vibration of the vibrator as B, and a vibration time of the vibrator as C, the control part may vibrate the vibrator based on the spray amount such that A = B × C is satisfied and the atomization rate is increased and the vibration time is shortened.

According to this embodiment, since it is possible to shorten the time of blowing air to the liquid in the mist form, it is possible to suppress degradation of the liquid.

For example, in the spray device according to an embodiment of the disclosure, the liquid may be hypochlorous acid water.

According to this embodiment, it is possible to suppress degradation of hypochlorous acid water.

For example, the spray device according to an embodiment of the disclosure may further include a control part which controls the spray part based on the liquid level detected by the detection part.

According to this embodiment, it is possible to control spray of the liquid based on the liquid level detected by the detection part.

For example, in the spray device according to an embodiment of the disclosure, the detection part may further detect at least one of a type and a state of the liquid held in the holding part. The control part may control the spray part based on at least one of the type and the state detected by the detection part.

According to this embodiment, it is possible to control spray of the liquid based on at least one of the type and the state of the liquid detected by the detection part.

To achieve the above objective, a control method according to an embodiment of the disclosure is a control method of a spray device. The spray device includes a holding part, a spray part, a first electrode, and a second electrode. The holding part is capable of holding a liquid inside the holding part. The spray part atomizes and sprays the liquid held in the holding part. The first electrode and the second electrode are provided inside the holding part. The control method includes a detection step of detecting a liquid level of the liquid held in the holding part. In the detection step, a first voltage is applied between the first electrode and the second electrode with the first electrode as an anode and the second electrode as a cathode, and after the first voltage is applied, a second voltage is applied between the first electrode and the second electrode with the first electrode as the cathode and the second electrode as the anode.

According to this embodiment, the same effect as the spray device described above is achieved.

In addition, the disclosure may be realized not only as a spray device including such a characteristic processing part, but also as a control method including steps of a process performed by the characteristic processing part included in the spray device. Further, the disclosure may also be realized as a program for causing a computer to function as the characteristic processing part included in the spray device or as a program that causes a computer to execute the characteristic steps included in the control method. Obviously, such a program may be distributed via a non-transitory computer-readable recording medium such as a compact disc-read only memory (CD-ROM) or a communication network such as the Internet.

### [Effect]

According to the spray device and the like according to an embodiment of the disclosure, it is possible to detect the liquid level of the liquid while suppressing deterioration of the electrodes.

### BRIEF DESCRIPTION OF THE DRAWINGS

FIG. 1 is a block diagram showing a functional configuration of a spray device according to a first embodiment.
FIG. 2 is a graph showing an example of potentials of a first electrode and a second electrode of the spray device in FIG. 1.
FIG. 3 is a graph showing another example of the potentials of the first electrode and the second electrode of the spray device in FIG. 1.
FIG. 4 is a graph showing still another example of the potentials of the first electrode and the second electrode of the spray device in FIG. 1.
FIG. 5 is a flowchart showing an example of an operation of the spray device in FIG. 1.
FIG. 6 is a flowchart showing an example of another operation of the spray device in FIG. 1.
FIG. 7 is a flowchart showing an example of still another operation of the spray device in FIG. 1.
FIG. 8 is a block diagram showing a functional configuration of a spray device according to a second embodiment.
FIG. 9 is a block diagram showing a functional configuration of a spray device according to a third embodiment.
FIG. 10 is a block diagram showing a functional configuration of a spray device according to a fourth embodiment.

### DESCRIPTION OF THE EMBODIMENTS

Hereinafter, embodiments of the disclosure will be described in detail with reference to the drawings. The embodiments described below all show comprehensive or specific examples. Numerical values, shapes, materials, components, arrangement positions and connections of the components, steps, the sequence of steps, etc. shown in the following embodiments are merely an example and are not intended to limit the disclosure. Further, among the components in the following embodiments, the components not described in the independent claims will be described as arbitrary components.

### (First embodiment)

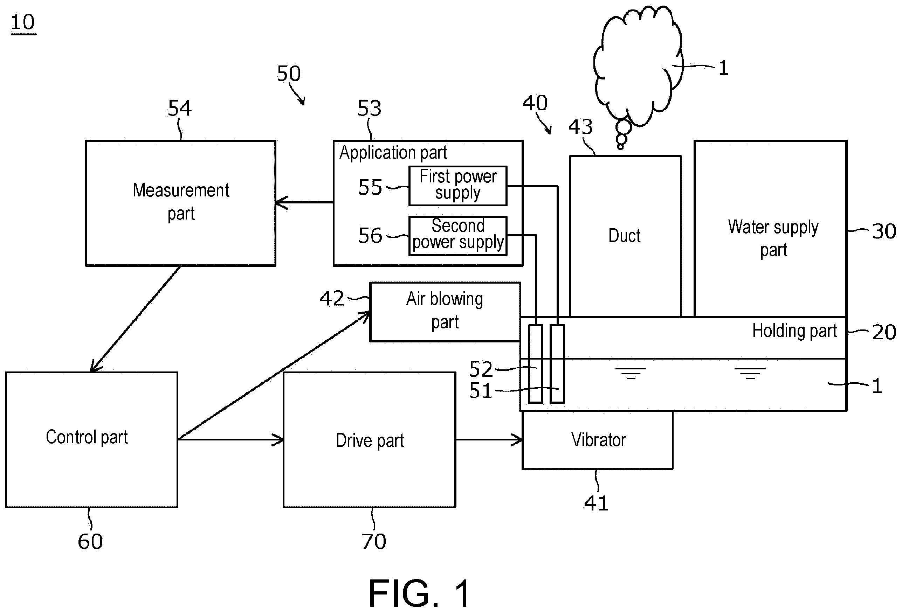

FIG. 1 is a block diagram showing a functional configuration of a spray device 10 according to a first embodiment.

The spray device 10 is a device that atomizes and sprays a liquid 1. For example, the liquid 1 is hypochlorous acid water, tap water, etc. For example, the spray device 10 is a humidifier. As shown in FIG. 1, the spray device 10 includes a holding part 20, a water supply part 30, a spray part 40, a detection part 50, a control part 60, and a drive part 70.

The holding part 20 is capable of holding the liquid 1 inside the holding part 20. In other words, the liquid 1 may be held inside the holding part 20. For example, the holding part 20 is a container capable of storing the liquid 1. Specifically, for example, the holding part 20 is a water tank.

The water supply part 30 holds the liquid 1 and supplies the held liquid 1 to the holding part 20. For example, the water supply part 30 is a water supply tank.

The spray part 40 atomizes and sprays the liquid 1 held in the holding part 20. In other words, the spray part 40 sprays the liquid 1 in a mist form. The spray part 40 includes a vibrator 41, an air blowing part 42, and a duct 43.

The vibrator 41 vibrates and atomizes the liquid 1. Specifically, with the vibrator 41 vibrating, the vibrator 41 vibrates and atomizes the liquid 1 held in the holding part 20. For example, the vibrator 41 is immersed in the liquid 1. The vibrator 41 may be provided at a position capable of vibrating and atomizing the liquid 1. For example, the vibrator 41 is an ultrasonic vibrator. Specifically, for example, the vibrator 41 is a piezoelectric vibrator.

The air blowing part 42 blows air to the liquid 1 atomized by the vibrator 41. In other words, the air blowing part 42 generates air to hit the liquid 1 atomized by the vibrator 41. The liquid 1 atomized by the vibrator 41 is carried away by the air generated by the air blowing part 42.

For example, the air blowing part 42 may also blow air to the vibrator 41. Accordingly, it is possible to suppress an increase in temperature of the vibrator 41. For example, in the case where the liquid 1 is hypochlorous acid water, it is possible to suppress an increase in temperature of the hypochlorous acid water and suppress acidification (deterioration) of the hypochlorous acid water.

The duct 43 discharges the liquid 1 atomized by the vibrator 41 to outside of the spray device 10. In this embodiment, the liquid 1 atomized by the vibrator 41 flows through the inside of the duct 43 with the air generated by the air blowing part 42 and is discharged from the duct 43 to outside of the spray device 10. In this manner, the spray device 10 sprays the liquid 1 in a mist form from the duct 43.

The detection part 50 detects a liquid level of the liquid 1 held in the holding part 20. Further, in this embodiment, the detection part 50 detects a type of the liquid held in the holding part 20. Further, in this embodiment, the detection part 50 detects a state of the liquid 1 held in the holding part 20. Further, in this embodiment, the detection part 50 includes a first electrode 51, a second electrode 52, an application part 53, and a measurement part 54.

The first electrode 51 and the second electrode 52 are provided inside the holding part 20. The first electrode 51 and the second electrode 52 are provided at a position at which a current value (resistance value) between the first electrode 51 and the second electrode 52 in the case where a voltage is applied between the first electrode 51 and the second electrode 52 changes according to the liquid level of the liquid 1 held in the holding part 20. For example, the first electrode 51 and the second electrode 52 are provided at a position immersed in the liquid 1 in the case where a predetermined amount or more of the liquid 1 is held in the holding part 20. The current value (resistance value) between the first electrode 51 and the second electrode 52 in the case where a voltage is applied between the first electrode 51 and the second electrode 52 changes as the liquid level of the liquid 1 held in the holding part 20 changes. Thus, the liquid level can be detected based on the current value (the resistance value). Further, for example, the current value (resistance value) between the first electrode 51 and the second electrode 52 in the case where a voltage is applied between the first electrode 51 and the second electrode 52 changes according to the type of the liquid 1 held in the holding part 20. Thus, the type of the liquid 1 can be detected based on the current value (the resistance value). Further, for example, the current value (resistance value) between the first electrode 51 and the second electrode 52 in the case where a voltage is applied between the first electrode 51 and the second electrode 52 changes according to the state of the liquid 1 held in the holding part 20. Thus, the state of the liquid 1 can be detected based on the current value (the resistance value). For example, each of the first electrode 51 and the second electrode 52 is an electrode rod.

The application part 53 applies a first voltage between the first electrode 51 and the second electrode 52 with the first electrode 51 as the anode and the second electrode 52 as the cathode, and then the application part 53 applies a second voltage between the first electrode 51 and the second electrode 52 with the first electrode 51 as the cathode and the second electrode 52 as the anode. The application part 53 includes a first power supply 55 and a second power supply 56.

The first power supply 55 is connected to the first electrode 51. The second power supply 56 is connected to the second electrode 52. In the case where the potential of the first electrode 51 is higher than the potential of the second electrode 52, the first electrode 51 becomes the anode, and the second electrode 52 becomes the cathode. In the case where the potential of the first electrode 51 is lower than the potential of the second electrode 52, the first electrode 51 becomes the cathode, and the second electrode 52 becomes the anode.

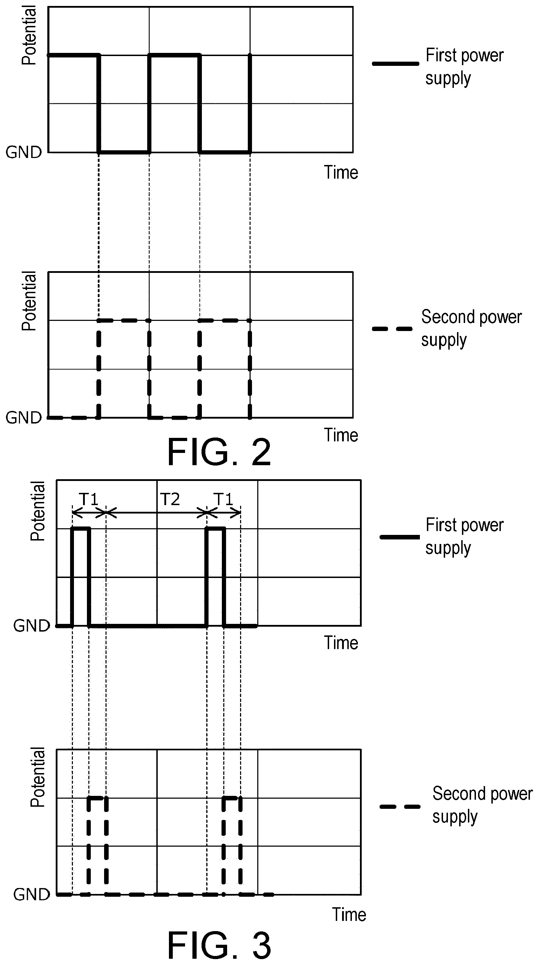

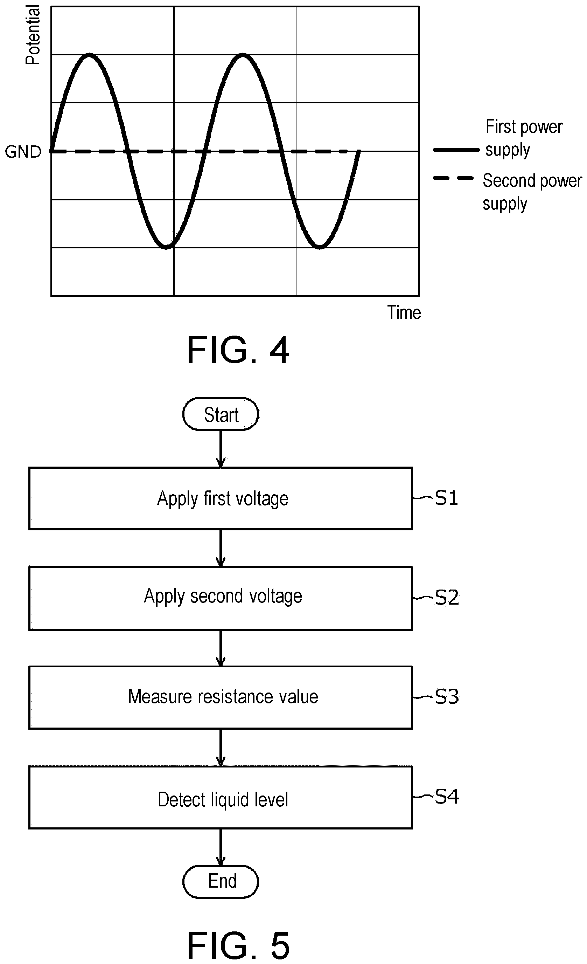

FIG. 2 is a graph showing an example of the potentials of the first electrode 51 and the second electrode 52 of the spray device 10 in FIG. 1. FIG. 3 is a graph showing another example of the potentials of the first electrode 51 and the second electrode 52 of the spray device 10 in FIG. 1. FIG. 4 is a graph showing still another example of the potentials of the first electrode 51 and the second electrode 52 of the spray device 10 in FIG. 1. In FIG. 2 to FIG. 4, the potential of the first electrode 51 is indicated by thick solid lines, and the potential of the second electrode 52 is indicated by thick broken lines.

As shown in FIG. 2 and FIG. 3, for example, when the first power supply 55 controls the potential of the first electrode 51 to be a potential higher than GND and the second power supply 56 controls the potential of the second electrode 52 to be GND, the first electrode 51 becomes the anode and the second electrode 52 becomes the cathode. Further, for example, when the first power supply 55 controls the potential of the first electrode 51 to be GND and the second power supply 56 controls the potential of the second electrode 52 to be a potential higher than GND, the first electrode 51 becomes the cathode and the second electrode 52 becomes the anode.

As shown in FIG. 4, for example, when the first power supply 55 controls the potential of the first electrode 51 to be a potential higher than GND and the second power supply 56 controls the potential of the second electrode 52 to be GND, the first electrode 51 becomes the anode and the second electrode 52 becomes the cathode. Further, for example, when the first power supply 55 controls the potential of the first electrode 51 to be a potential lower than GND and the second power supply 56 controls the potential of the second electrode 52 to be GND, the first electrode 51 becomes the cathode and the second electrode 52 becomes the anode.

For example, the application part 53 applies the second voltage such that the waveform of the second voltage is an inverted waveform of the waveform of the first voltage.

As shown in FIG. 2 and FIG. 3, for example, the magnitude and waveform of the potential of the first electrode 51 over a time in which the first electrode 51 is the anode and the second electrode 52 is the cathode are made consistent with the magnitude and waveform of the potential of the second electrode 52 over a time in which the first electrode 51 is the cathode and the second electrode 52 is the anode. Also, the magnitude and waveform of the potential of the second electrode 52 over a time in which the first electrode 51 is the anode and the second electrode 52 is the cathode are made consistent with the magnitude and waveform of the potential of the first electrode 51 over a time in which the first electrode 51 is the cathode and the second electrode 52 is the anode. Accordingly, the waveform of the second voltage becomes an inverted waveform of the waveform of the first voltage.

As shown in FIG. 4, for example, in the case where the potential of the second electrode 52 is set to GND and only the potential of the first electrode 51 is changed, the waveform of the potential of the first electrode 51 over a time in which the first electrode 51 is the cathode and the second electrode 52 is the anode is set to be a waveform obtained by inverting, with respect to GND as the reference, the waveform of the potential of the first electrode 51 over a time in which the first electrode 51 is the anode and the second electrode 52 is the cathode. Accordingly, the waveform of the second voltage becomes an inverted waveform of the waveform of the first voltage.

For example, the application part 53 intermittently performs an operation of applying the first voltage and then applying the second voltage. In other words, for example, the application part 53 applies the first voltage and then applies the second voltage, and subsequently, after a time interval, the application part 53 applies the first voltage and then applies the second voltage.

As shown in FIG. 3, for example, after the first power supply 55 controls the potential of the first electrode 51 to be a potential higher than GND and the second power supply 56 controls the potential of the second electrode 52 to be GND, the first power supply 55 controls the potential of the first electrode 51 to be GND and the second power supply 56 controls the potential of the second electrode 52 to be a potential higher than GND. Subsequently, the first power supply 55 controls the potential of the first electrode 51 to be GND and the second power supply 56 controls the potential of the second electrode 52 to be GND. Subsequently, again, after the first power supply 55 controls the potential of the first electrode 51 to be a potential higher than GND and the second power supply 56 controls the potential of the second electrode 52 to be GND, the first power supply 55 controls the potential of the first electrode 51 to be GND and the second power supply 56 controls the potential of the second electrode 52 to be a potential higher than GND. For example, by doing so, the first voltage is applied and then the second voltage is applied, and subsequently, after a time interval, the first voltage is applied and then the second voltage is applied.

Furthermore, for example, as shown in FIG. 2 and FIG. 4, the application part 53 may also continuously perform the operation of applying the first voltage and then applying the second voltage.

For example, the application part 53 applies the first voltage and the second voltage in succession.

As shown in FIG. 2 and FIG. 3, for example, the first power supply 55 continuously changes the potential of the first electrode 51 between a potential higher than GND and GND, and the second power supply 56 continuously changes the potential of the second electrode 52 between GND and a potential higher than GND. Accordingly, the first voltage and the second voltage are applied in succession.

As shown in FIG. 4, for example, the second power supply 56 keeps the potential of the second electrode 52 at GND, and the first power supply 55 continuously changes the potential of the first electrode 51 between a potential higher than GND and a potential lower than GND. Accordingly, the first voltage and the second voltage are applied in succession.

For example, the application part 53 intermittently performs the operation of applying the first voltage and the second voltage in succession at a time interval longer than a time of applying the first voltage and the second voltage in succession. In other words, for example, the application part 53 applies the first voltage and the second voltage in succession, and subsequently, after a time interval longer than the time of applying the first voltage and the second voltage in succession, the application part 53 applies the first voltage and the second voltage in succession.

As shown in FIG. 3, for example, over a time T1, the first power supply 55 continuously changes the potential of the first electrode 51 between a potential higher than GND and GND, and the second power supply 56 continuously changes the potential of the second electrode 52 between GND and a potential higher than GND. Accordingly, the first voltage and the second voltage are applied in succession. Subsequently, over a time T2 longer than the time T1, the first power supply 55 controls the potential of the first electrode 51 to be GND, and the second power supply 56 controls the potential of the second electrode 52 to be GND. Accordingly, the first voltage and the second voltage are not applied. Subsequently, again, over a time T1, the first power supply 55 continuously changes the potential of the first electrode 51 between a potential higher than GND and GND, and the second power supply 56 continuously changes the potential of the second electrode 52 between GND and a potential higher than GND. Accordingly, the first voltage and the second voltage are applied in succession. For example, by doing so, the first voltage and the second voltage are applied in succession, and subsequently, after a time interval longer than the time of applying the first voltage and the second voltage in succession, the first voltage and the second voltage are applied in succession. For example, during the time T1, a time over which the potential of the first electrode 51 is higher than GND and the potential of the second electrode 52 is GND is equal to a time over which the potential of the first electrode 51 is GND and the potential of the second electrode 52 is higher than GND.

Furthermore, for example, as shown in FIG. 2 and FIG. 4, the application part 53 may continuously perform the operation of applying the first voltage and the second voltage in succession.

For example, the application part 53 does not apply the first voltage and the second voltage when the liquid is not being sprayed by the spray part 40. Specifically, for example, the application part 53 does not apply the first voltage and the second voltage when the vibrator 41 itself is not vibrating and the vibrator 41 is not vibrating the liquid 1.

Returning to FIG. 1, the measurement part 54 measures at least one of a current value and a resistance value between the first electrode 51 and the second electrode 52 in the case where a voltage is applied between the first electrode 51 and the second electrode 52. Specifically, for example, the measurement part 54 measures at least one of the current value and the resistance value between the first electrode 51 and the second electrode 52 in the case where the first voltage is applied between the first electrode 51 and the second electrode 52. Further, for example, the measurement part 54 measures at least one of the current value and the resistance value between the first electrode 51 and the second electrode 52 in the case where the second voltage is applied between the first electrode 51 and the second electrode 52. For example, the measurement part 54 may be an ammeter and measures a current value. Further, for example, the measurement part 54 may be a resistance meter and measures a resistance value. In addition, for example, the measurement part 54 may measure an impedance.

In this embodiment, in the case where a voltage is applied between the first electrode 51 and the second electrode 52, the measurement part 54 measures a voltage at a resistor end of a fixed resistor (not shown) provided between the first power supply 55 and the first electrode 51 to measure a current value (resistance value) between the first electrode 51 and the second electrode 52. In this embodiment, the fixed resistor is built into the application part 53.

The detection part 50 measures at least one of a current value and a resistance value using the first electrode 51 and the second electrode 52, and detects a liquid level of the liquid 1 held in the holding part 20 based on at least one of the current value and the resistance value. For example, the detection part 50 detects whether the liquid level of the liquid 1 held in the holding part 20 is equal to or higher than a predetermined height by comparing at least one of the current value and the resistance value with a threshold value.

Further, the detection part 50 measures at least one of a current value and a resistance value using the first electrode 51 and the second electrode 52, and detects a type of the liquid 1 held in the holding part 20 based on at least one of the current value and the resistance value. For example, the detection part 50 detects whether the type of the liquid 1 held in the holding part 20 is hypochlorous acid water or not, whether it is tap water or not, etc., by comparing at least one of the current value and the resistance value with a threshold value.

Further, the detection part 50 measures at least one of a current value and a resistance value using the first electrode 51 and the second electrode 52, and detects a state of the liquid 1 held in the holding part 20 based on at least one of the current value and the resistance value. For example, in the case where the liquid 1 is hypochlorous acid water, the detection part 50 detects whether a concentration of the liquid 1 held in the holding part 20 is equal to or greater than a predetermined concentration by comparing at least one of the current value and the resistance value with a threshold value.

The control part 60 controls the spray part 40.

For example, the control part 60 controls the spray part 40 based on the liquid level of the liquid 1 detected by the detection part 50. Specifically, for example, the control part 60 controls the vibrator 41 and the air blowing part 42 based on the liquid level of the liquid 1 detected by the detection part 50. For example, in the case where the liquid level of the liquid 1 detected by the detection part 50 is lower than the predetermined height, the control part 60 controls the spray part 40 so as not to spray the liquid 1. Specifically, for example, in the case where the liquid level of the liquid 1 detected by the detection part 50 is lower than the predetermined height, the control part 60 controls the vibrator 41 such that the vibrator 41 does not vibrate, and controls the air blowing part 42 such that the air blowing part 42 does not blow air.

For example, the control part 60 drives the air blowing part 42 in conjunction with the vibrator 41. For example, the control part 60 drives the air blowing part 42 when the vibrator 41 is being vibrated, and the control part 60 does not drive the air blowing part 42 when the vibrator 41 is not being vibrated. In this manner, for example, the control part 60 stops air blowing of the air blowing part 42 when vibration of the vibrator 41 is stopped.

For example, taking a spray amount of the liquid 1 required to be sprayed as A, an atomization rate of the liquid 1 due to vibration of the vibrator 41 as B, and a vibration time of the vibrator 41 as C, the control part 60 vibrates the vibrator 41 based on the spray amount such that A = B × C is satisfied and the atomization rate is increased and the vibration time is shortened.

For example, the spray amount of the liquid 1 required to be sprayed is a spray amount of the liquid 1 required to be sprayed per predetermined time, and is inputted by a user or the like.

For example, in the case where the spray amount of the liquid 1 required to be sprayed is 100 mL, the atomization rate in the case of vibrating the vibrator 41 at a first vibration frequency is 400 mL/h, and the atomization rate in the case of vibrating the vibrator 41 at a second vibration frequency is 100 mL/h, and it is possible to select whether to vibrate the vibrator 41 at the first vibration frequency or the second vibration frequency, the control part 60 vibrates the vibrator 41 at the first vibration frequency for 15 minutes, such that A = B × C is satisfied, and the atomization rate is increased and the vibration time is shortened. For example, the atomization rate of the liquid 1 is measured in advance and stored in the spray device 10.

Further, the control part 60 controls the spray part 40 based on at least one of the type of the liquid 1 and the state of the liquid 1 detected by the detection part 50.

For example, in the case where the type of the liquid 1 detected by the detection part 50 is hypochlorous acid water, the control part 60 controls the spray part 40 such that the spray amount per unit time does not exceed a predetermined amount. Further, for example, in the case where the type of the liquid 1 detected by the detection part 50 is salt water or bleach, the control part 60 controls the spray part 40 so as not to spray the liquid 1. Further, for example, in the case where the type of the liquid 1 detected by the detection part 50 is hypochlorous acid water and the concentration of the hypochlorous acid water is lower than the predetermined concentration, the control part 60 controls the spray part 40 so as not to spray the liquid 1.

The drive part 70 drives the vibrator 41. For example, the drive part 70 is a circuit that vibrates the vibrator 41.

FIG. 5 is a flowchart showing an example of an operation of the spray device 10 in FIG. 1. The operation includes a detection step (steps S1 to S4) of detecting the liquid level of the liquid 1 held in the holding part 20.

In the detection step, first, taking the first electrode 51 as the anode and the second electrode 52 as the cathode, the application part 53 applies a first voltage between the first electrode 51 and the second electrode 52 (step S1). Specifically, for example, as described above, the application part 53 applies the first voltage.

After applying the first voltage, taking the first electrode 51 as the cathode and the second electrode 52 as the anode, the application part 53 applies a second voltage between the first electrode 51 and the second electrode 52 (step S2). Specifically, for example, as described above, the application part 53 applies the second voltage.

The measurement part 54 measures a resistance value (step S3). For example, the measurement part 54 measures a resistance value between the first electrode 51 and the second electrode 52 in the case where the first voltage is applied between the first electrode 51 and the second electrode 52. Further, for example, the measurement part 54 measures a resistance value between the first electrode 51 and the second electrode 52 in the case where the second voltage is applied between the first electrode 51 and the second electrode 52. For example, as described above, the measurement part 54 measures the resistance value between the first electrode 51 and the second electrode 52. In addition, for example, the measurement part 54 may measure a current value instead of the resistance value.

The detection part 50 detects a liquid level of the liquid 1 held in the holding part 20 (step S4).

For example, the liquid 1 is hypochlorous acid water, and with the liquid level of the liquid 1 held in the holding part 20 having dropped to a predetermined liquid level, a resistance value between the first electrode 51 and the second electrode 52 in the case where a voltage is applied between the first electrode 51 and the second electrode 52 is measured in advance as a first threshold value. With the inside of the holding part 20 being fully filled with the liquid 1, a resistance value between the first electrode 51 and the second electrode 52 in the case where a voltage is applied between the first electrode 51 and the second electrode 52 is measured in advance as a second threshold value. The first threshold value and the second threshold value are stored in the spray device 10.

For example, the detection part 50 detects that the liquid level of the liquid 1 held in the holding part 20 is the predetermined liquid level or higher in the case where the measured resistance value is equal to or less than the first threshold value. Further, for example, the detection part 50 detects that the liquid level of the liquid 1 held in the holding part 20 has dropped below the predetermined liquid level in the case where the measured resistance value is greater than the first threshold value.

For example, the detection part 50 further detects at least one of the type and the state of the liquid 1 held in the holding part 20. For example, in the case where the measured resistance value is less than the second threshold value, the detection part 50 detects that the type of the liquid 1 held in the holding part 20 is a water incompatible with the spray device 10, or that the state of the liquid 1 held in the holding part 20 is abnormal. Further, for example, in the case where the measured resistance value is equal to or greater than the second threshold value and equal to or less than the first threshold value, the detection part 50 detects that the type of the liquid 1 held in the holding part 20 is hypochlorous acid water, and that the state of the liquid 1 held in the holding part 20 is normal.

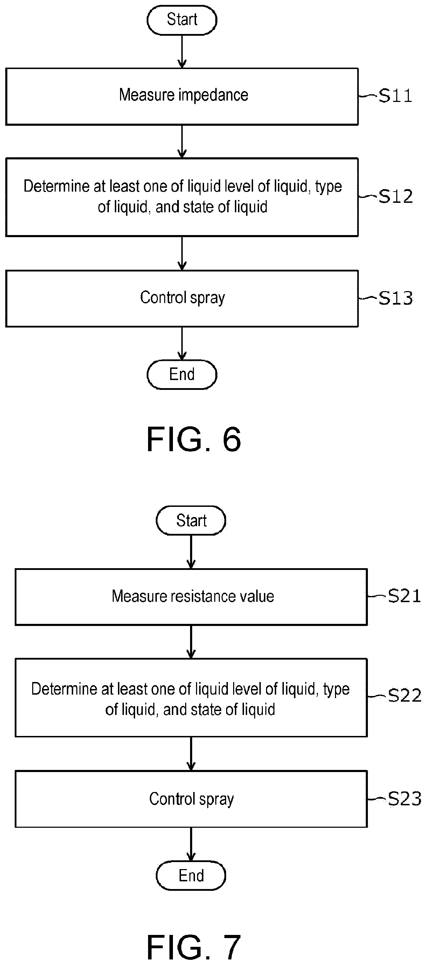

FIG. 6 is a flowchart showing an example of another operation of the spray device 10 in FIG. 1.

As shown in FIG. 6, the measurement part 54 measures an impedance (step S11). For example, the measurement part 54 measures an impedance between the first electrode 51 and the second electrode 52 in the case where the first voltage is applied between the first electrode 51 and the second electrode 52. Further, for example, the measurement part 54 measures an impedance between the first electrode 51 and the second electrode 52 in the case where the second voltage is applied between the first electrode 51 and the second electrode 52.

The control part 60 determines at least one of a liquid level of the liquid 1, a type of the liquid 1, and a state of the liquid 1 held in the holding part 20 based on the impedance measured by the measurement part 54 (step S12). Specifically, the control part 60 determines at least one of the liquid level of the liquid 1, the type of the liquid 1, and the state of the liquid 1 held in the holding part 20 based on a value of the impedance measured by the measurement part 54.

The control part 60 controls the spray part 40 based on at least one of the liquid level of the liquid 1, the type of the liquid 1, and the state of the liquid 1 held in the holding part 20 (step S13).

For example, in the case where the type of the liquid 1 held in the holding part 20 is hypochlorous acid water, the control part 60 sprays the liquid 1 from the spray part 40 while managing the spray amount.

For example, in the case where the type of the liquid 1 held in the holding part 20 is tap water, the control part 60 constantly sprays the liquid 1 from the spray part 40 and constantly drives the vibrator 41 and the air blowing part 42. Further, for example, in the case where the type of the liquid 1 held in the holding part 20 is tap water and the spray device 10 is a device incapable of using tap water, the control part 60 does not drive the spray part 40.

Further, for example, in the case where there is no liquid 1 held in the holding part 20, the control part 60 does not drive the spray part 40. Specifically, for example, in the case where there is no liquid 1 held in the holding part 20, the control part 60 does not drive the vibrator 41 and the air blowing part 42. For example, in this case, the control part 60 displays a request for water supply to the holding part 20 on a monitor (not shown) or the like.

Further, for example, in the case where the type of the liquid 1 held in the holding part 20 is hypochlorous acid water and the state of the liquid 1 is poor (e.g., in the case where its efficacy has decreased due to being left for a long time), the control part 60 does not drive the spray part 40. In this case, for example, the control part 60 displays a warning and a request for replacing the liquid 1 held in the holding part 20 on a monitor (not shown) or the like.

Further, for example, in the case where the type of the liquid 1 held in the holding part 20 is salt water or bleach, the control part 60 does not drive the spray part 40. For example, in this case, the control part 60 displays a warning and a request for replacing the liquid 1 held in the holding part 20 on a monitor (not shown) or the like.

FIG. 7 is a flowchart showing an example of another operation of the spray device 10 in FIG. 1.

As shown in FIG. 7, the measurement part 54 measures a resistance value (step S21). For example, the measurement part 54 measures a resistance value between the first electrode 51 and the second electrode 52 in the case where the first voltage is applied between the first electrode 51 and the second electrode 52. Further, for example, the measurement part 54 measures a resistance value between the first electrode 51 and the second electrode 52 in the case where the second voltage is applied between the first electrode 51 and the second electrode 52.

The control part 60 determines at least one of a liquid level of the liquid 1, a type of the liquid 1, and a state of the liquid 1 held in the holding part 20 based on the resistance value measured by measurement part 54 (step S22).

The control part 60 controls the spray part 40 based on at least one of the liquid level of the liquid 1, the type of the liquid 1, and the state of the liquid 1 held in the holding part 20 (step S23).

For example, in the case where the resistance value is 20 kQ or less, the control part 60 determines that the type of the liquid 1 is incompatible with the spray device 10 and does not drive the spray part 40. For example, in this case, the control part 60 displays a request for replacing the liquid 1 held in the holding part 20 on a monitor (not shown) or the like.

For example, in the case where the resistance value is greater than 20 kQ and less than 500 kQ, the control part 60 determines that the liquid 1 is normal hypochlorous acid water and drives the spray part 40.

For example, in the case where the resistance value is 500 kQ or more, the control part 60 determines that the liquid 1 is not held in the holding part 20 and does not drive the spray part 40. For example, in this case, the control part 60 displays a request for water supply to the holding part 20 on a monitor (not shown) or the like.

As described above, in the spray device 10, since the liquid level of the liquid 1 held in the holding part 20 can be detected, it is possible to suppress vibration of the vibrator 41 and suppress damage of the vibrator 41 in the case where the liquid 1 is not held in the holding part 20.

Further, in the spray device 10, since the first voltage is applied and then the second voltage is applied, it is possible to suppress occurrence of electrolytic corrosion on the first electrode 51 and the second electrode 52 and melting of the first electrode 51 and the second electrode 52, and it is possible to suppress mixing of melted metal into the liquid 1. Accordingly, in the case where the liquid 1 is hypochlorous acid water, it is possible to suppress a decrease in the efficacy of the hypochlorous acid water.

A spray device 10 according to this embodiment includes a holding part 20, a spray part 40, and a detection part 50. The holding part 20 is capable of holding a liquid 1 inside the holding part 20. The spray part 40 atomizes and sprays the liquid 1 held in the holding part 20. The detection part 50 detects a liquid level of the liquid 1 held in the holding part 20. The detection part 50 includes a first electrode 51, a second electrode 52, and an application part 53. The first electrode 51 and the second electrode 52 are provided inside the holding part 20. The application part 53 applies a first voltage between the first electrode 51 and the second electrode 52 with the first electrode 51 as an anode and the second electrode 52 as a cathode, and then applies a second voltage between the first electrode 51 and the second electrode 52 with the first electrode 51 as the cathode and the second electrode 52 as the anode.

According to this embodiment, with the detection part 50 which detects the liquid level of the liquid 1 held in the holding part 20, it is possible to detect the liquid level of the liquid 1. Further, since the detection part 50 includes the application part 53 which applies the first voltage between the first electrode 51 and the second electrode 52 with the first electrode 51 as the anode and the second electrode 52 as the cathode, and then applies the second voltage between the first electrode 51 and the second electrode 52 with the first electrode 51 as the cathode and the second electrode 52 as the anode, it is possible to suppress deterioration of the electrodes. In this manner, it is possible to detect the liquid level of the liquid 1 while suppressing deterioration of the electrodes.

Further, in the spray device 10 according to this embodiment, the application part 53 applies the second voltage such that a waveform of the second voltage is an inverted waveform of a waveform of the first voltage.

According to this embodiment, it is possible to detect the liquid level of the liquid 1 while further suppressing deterioration of the electrodes.

Further, in the spray device 10 according to this embodiment, the application part 53 intermittently performs an operation of applying the first voltage and then applying the second voltage.

According to this embodiment, it is possible to repeatedly detect the liquid level of the liquid 1 while further suppressing deterioration of the electrodes.

Further, in the spray device 10 according to this embodiment, the application part 53 applies the first voltage and the second voltage in succession.

According to this embodiment, it is possible to detect the liquid level of the liquid 1 while further suppressing deterioration of the electrodes.

Further, in the spray device 10 according to this embodiment, the application part 53 intermittently performs an operation of applying the first voltage and the second voltage in succession at a time interval longer than a time of applying the first voltage and the second voltage in succession.

According to this embodiment, it is possible to repeatedly detect the liquid level of the liquid 1 while further suppressing deterioration of the electrodes.

Further, in the spray device 10 according to this embodiment, the application part 53 does not apply the first voltage and the second voltage in a case where the liquid 1 is not being sprayed by the spray part 40.

According to this embodiment, it is possible to detect the liquid level of the liquid 1 while further suppressing deterioration of the electrodes.

Further, in the spray device 10 according to this embodiment, the spray part 40 includes a vibrator 41 which vibrates and atomizes the liquid 1, and an air blowing part 42 which blows air to the liquid 1 atomized by the vibrator 41.

According to this embodiment, it is possible to detect the liquid level of the liquid 1 while suppressing deterioration of the electrodes, and it is possible to easily spray the liquid 1 held in the holding part 20.

Further, the spray device 10 according to this embodiment further includes a control part 60 which controls the spray part 40. The control part 60 drives the air blowing part 42 in conjunction with the vibrator 41.

According to this embodiment, since the air blowing part 42 can be driven when the vibrator 41 is vibrating, it is possible to efficiently spray the liquid 1 held in the holding part 20.

Further, in the spray device 10 according to this embodiment, the control part 60 stops air blowing of the air blowing part 42 in a case where vibration of the vibrator 41 has stopped.

According to this embodiment, it is possible to suppress air blowing of the air blowing part 42 when the liquid 1 is not atomized and air blowing is not required.

Further, in the spray device 10 according to this embodiment, taking a spray amount of the liquid 1 required to be sprayed as A, an atomization rate of the liquid 1 due to vibration of the vibrator 41 as B, and a vibration time of the vibrator 41 as C, the control part 60 vibrates the vibrator 41 based on the spray amount such that A = B × C is satisfied and the atomization rate is increased and the vibration time is shortened.

According to this embodiment, since it is possible to shorten the time of blowing air to the liquid 1 in the mist form, it is possible to suppress degradation of the liquid 1.

Further, in the spray device 10 according to this embodiment, the liquid 1 is hypochlorous acid water.

According to this embodiment, it is possible to suppress degradation of hypochlorous acid water.

Further, the spray device 10 according to this embodiment further includes a control part 60 which controls the spray part 40 based on the liquid level detected by the detection part 50.

According to this embodiment, it is possible to control spray of the liquid 1 based on the liquid level detected by the detection part 50.

Further, in the spray device 10 according to this embodiment, the detection part 50 further detects at least one of a type and a state of the liquid 1 held in the holding part 20. The control part 60 controls the spray part 40 based on at least one of the type and the state detected by the detection part 50.

According to this embodiment, it is possible to control spray of the liquid 1 based on at least one of the type and the state of the liquid 1 detected by the detection part 50.

A control method according to an embodiment is a control method of a spray device 10. The spray device 10 includes a holding part 20, a spray part 40, a first electrode 51, and a second electrode 52. The holding part 20 is capable of holding a liquid 1 inside the holding part 20. The spray part 40 atomizes and sprays the liquid 1 held in the holding part 20. The first electrode 51 and the second electrode 52 are provided inside the holding part 20. The control method includes a detection step of detecting a liquid level of the liquid 1 held in the holding part 20. In the detection step, a first voltage is applied between the first electrode 51 and the second electrode 52 with the first electrode 51 as an anode and the second electrode 52 as a cathode (step S1), and after the first voltage is applied, a second voltage is applied between the first electrode 51 and the second electrode 52 with the first electrode 51 as the cathode and the second electrode 52 as the anode (step S2).

According to this embodiment, the same effect as the spray device 10 described above is achieved.

### (Second embodiment)

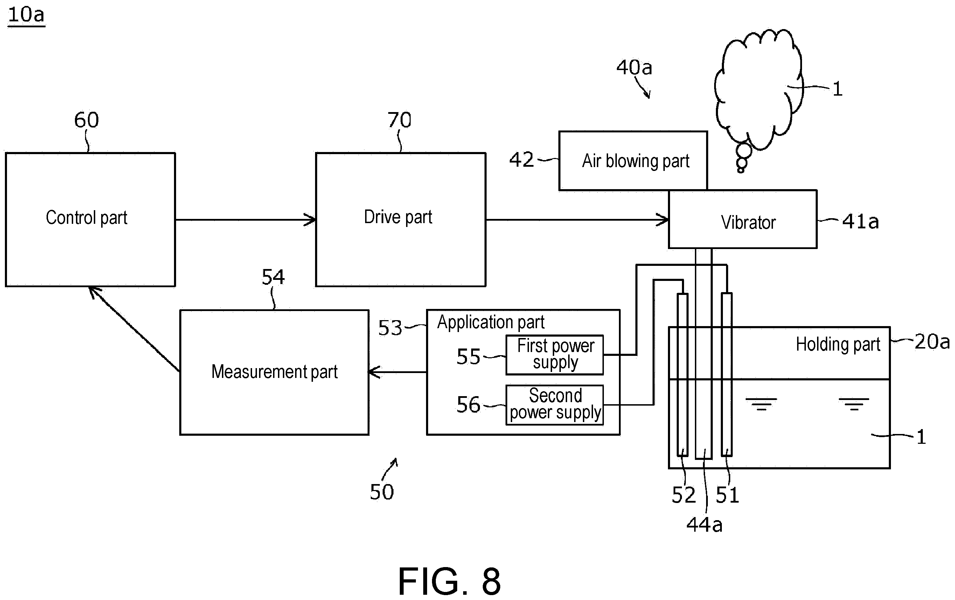

FIG. 8 is a block diagram showing a functional configuration of a spray device 10a according to a second embodiment.

The spray device 10a includes a holding part 20a, a spray part 40a, a detection part 50, a control part 60, and a drive part 70.

The spray part 40a includes a vibrator 41a, an air blowing part 42, and a water suction part 44a.

A portion of the water suction part 44a is immersed in the liquid 1 held in the holding part 20a, and the water suction part 44a sucks up the liquid 1 held in the holding part 20a. For example, the water suction part 44a is a tubular member.

The vibrator 41a is connected to the water suction part 44a, vibrates and atomizes the liquid 1 sucked up by the water suction part 44a, and discharges the atomized liquid 1. For example, the vibrator 41a vibrates and atomizes the liquid 1 inside the vibrator 41a, and discharges the liquid in a mist form from a hole formed at the vibrator 41a.

### (Third embodiment)

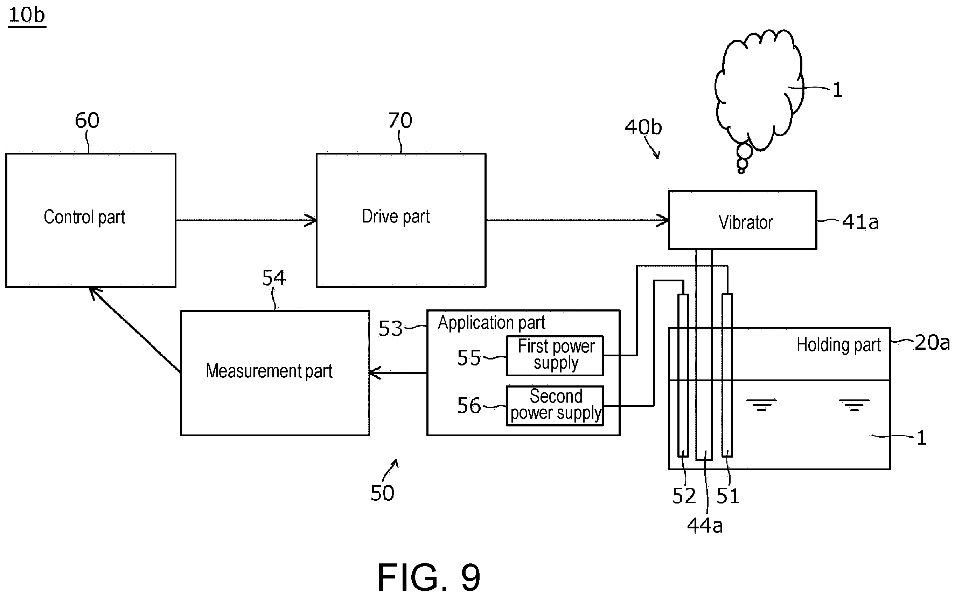

FIG. 9 is a block diagram showing a functional configuration of a spray device 10b according to a third embodiment.

The spray device 10b mainly differs from the spray device 10a in that the spray device 10b includes a spray part 40b instead of the spray part 40a.

The spray part 40b mainly differs from the spray part 40a in that the spray part 40b does not include the air blowing part 42.

### (Fourth embodiment)

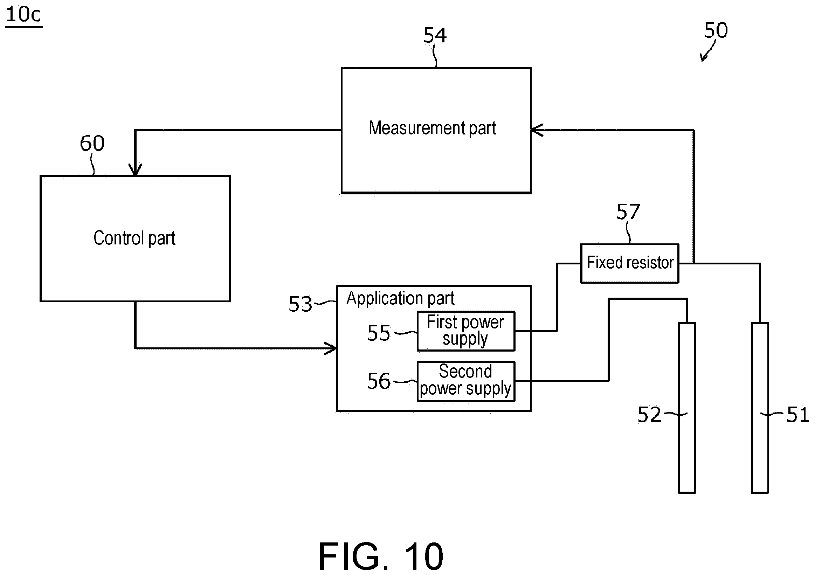

FIG. 10 is a block diagram showing a functional configuration of a spray device 10c according to a fourth embodiment. In FIG. 10, illustration of the holding part 20 and the like is omitted.

The spray device 10c mainly differs from the spray device 10 in that a fixed resistor 57 is provided outside the application part 53.

The measurement part 54 measures at least one of a resistance value and a current value between the first electrode 51 and the second electrode 52 by measuring a voltage across two ends of the fixed resistor 57.

The control part 60 controls the application part 53 based on a measurement result of the measurement part 54.

### (Other embodiments)

Although the spray device according to the embodiments of the disclosure has been described above, the disclosure is not limited to the above embodiments.

Specifically, each of the above devices may be configured as a computer system composed of a microprocessor, a ROM, a RAM, a hard disk drive, a display unit, a keyboard, a mouse, and the like. Computer programs are stored in the RAM or the hard disk drive. Each device achieves its function by operation of the microprocessor according to the computer program. Herein, the computer program is configured by combining a plurality of instruction codes indicating commands to a computer in order to achieve a predetermined function.

Further, some or all of the components that form each of the above devices may be composed of one system large-scale integration (LSI). A system LSI is a super-multifunctional LSI manufactured by integrating a plurality of components on one chip, and, for example, includes a computer system configured to include a microprocessor, a ROM, a RAM, and the like. In this case, the computer program is stored in the ROM. The system LSI achieves its function by operation of the microprocessor according to the computer program.

Furthermore, some or all of the components forming each of the above devices may be composed of an IC card or a separate module that may be attached to and detached from each device. The IC card or the module is a computer system composed of a microprocessor, a ROM, a RAM, and the like. The IC card or the module may include the above super-multifunctional LSI. The IC card or the module achieves its function by operation of the microprocessor according to the computer program. The IC card or the module may have tamper resistance.

Further, the disclosure may be the methods shown above. Further, the disclosure may be computer programs that realize these methods by a computer, or may be digital signals composed of the above computer programs.

Further, the disclosure may record the above computer program or the above digital signal to a non-transitory computer-readable recording medium, such as a flexible disk, a hard disk, a CD-ROM, an MO, a DVD, a DVD-ROM, a DVD-RAM, a BD (Blu-ray (registered trademark) disc), a semiconductor memory, or the like. Further, it may be the digital signal recorded on these non-transitory recording media.

Further, the disclosure may transmit the computer program or the digital signal via a telecommunication line, a wireless or wired communication line, a network represented by the Internet, data broadcasting, or the like.

Further, the disclosure may be a computer system including a microprocessor and a memory, the memory may store the above computer program, and the microprocessor may operate according to the computer program.

Further, by recording the programs or the digital signals on the non-transitory recording medium and transferring the same, or by transferring the programs or the digital signals via the network or the like, they may be executed by another independent computer system.

Further, the above embodiments and the above modification examples may be combined with each other.

The spray device according to the disclosure may be applied as a device that sprays a liquid in a mist form.

### Reference Signs List

10, 10a, 10b, 10c Spray device
20, 20a Holding part
30 Water supply part
40, 40a, 40b Spray part
41, 41a Vibrator
42 Air blowing part
43 Duct
44a Water suction part
50 Detection part
51 First electrode
52 Second electrode
53 Application part
54 Measurement part
55 First power supply
56 Second power supply
57 Fixed resistor
60 Control part
70 Drive part

## Claims

1. A spray device (10, 10a, 10b, 10c) comprising:
a holding part (20, 20a) capable of holding a liquid inside the holding part (20, 20a);
a spray part (40, 40a, 40b) which atomizes and sprays the liquid held in the holding part (20, 20a); and
a detection part (50) which detects a liquid level of the liquid held in the holding part (20, 20a), wherein
the detection part (50) comprises:
a first electrode (51) and a second electrode (52) provided inside the holding part (20, 20a); and
an application part (53) which applies a first voltage between the first electrode (51) and the second electrode (52) with the first electrode (51) as an anode and the second electrode (52) as a cathode, and then applies a second voltage between the first electrode (51) and the second electrode (52) with the first electrode (51) as the cathode and the second electrode (52) as the anode.

2. The spray device (10, 10a, 10b, 10c) according to claim 1, wherein
the application part (53) applies the second voltage such that a waveform of the second voltage is an inverted waveform of a waveform of the first voltage.

3. The spray device (10, 10a, 10b, 10c) according to claim 1, wherein
the application part (53) intermittently performs an operation of applying the first voltage and then applying the second voltage.

4. The spray device (10, 10a, 10b, 10c) according to claim 1, wherein
the application part (53) applies the first voltage and the second voltage in succession.

5. The spray device (10, 10a, 10b, 10c) according to claim 4, wherein
the application part (53) intermittently performs an operation of applying the first voltage and the second voltage in succession at a time interval longer than a time of applying the first voltage and the second voltage in succession.

6. The spray device (10, 10a, 10b, 10c) according to any one of claims 1 to 5, wherein
the application part (53) does not apply the first voltage and the second voltage in a case where the liquid is not being sprayed by the spray part (40, 40a, 40b).

7. The spray device (10, 10a, 10c) according to claim 1, wherein
the spray part (40, 40a) comprises a vibrator (41, 41a) which vibrates and atomizes the liquid, and an air blowing part (42) which blows air to the liquid atomized by the vibrator (41, 41a).

8. The spray device (10, 10a, 10b, 10c) according to claim 7, further comprising a control part (60) which controls the spray part (40, 40a), wherein
the control part (60) drives the air blowing part (42) in conjunction with the vibrator (41, 41a).

9. The spray device (10, 10a, 10b, 10c) according to claim 8, wherein
the control part (60) stops air blowing of the air blowing part (42) in a case where vibration of the vibrator (41, 41a) has stopped.

10. The spray device (10, 10a, 10b, 10c) according to claim 8 or 9, wherein
taking a spray amount of the liquid required to be sprayed as A, an atomization rate of the liquid due to vibration of the vibrator (41, 41a) as B, and a vibration time of the vibrator (41, 41a) as C, the control part (60) vibrates the vibrator (41, 41a) based on the spray amount such that A = B × C is satisfied and the atomization rate is increased and the vibration time is shortened.

11. The spray device (10, 10a, 10b, 10c) according to claim 1, wherein
the liquid is hypochlorous acid water.

12. The spray device (10, 10a, 10b, 10c) according to claim 1, further comprising a control part (60) which controls the spray part (40, 40a, 40b) based on the liquid level detected by the detection part (50).

13. The spray device (10, 10a, 10b, 10c) according to claim 12, wherein
the detection part (50) further detects at least one of a type and a state of the liquid held in the holding part (20, 20a), and
the control part (60) controls the spray part (40, 40a, 40b) based on at least one of the type and the state detected by the detection part (50).

14. A control method, which is a control method of a spray device (10, 10a, 10b, 10c), the spray device (10, 10a, 10b, 10c) comprising:
a holding part (20, 20a) capable of holding a liquid inside the holding part (20, 20a);
a spray part (40, 40a, 40b) which atomizes and sprays the liquid held in the holding part (20, 20a); and
a first electrode (51) and a second electrode (52) provided inside the holding part (20, 20a),
the control method comprising:
a detection step of detecting a liquid level of the liquid held in the holding part (20, 20a), wherein
in the detection step,
a first voltage is applied between the first electrode (51) and the second electrode (52) with the first electrode (51) as an anode and the second electrode (52) as a cathode, and
after the first voltage is applied, a second voltage is applied between the first electrode (51) and the second electrode (52) with the first electrode (51) as the cathode and the second electrode (52) as the anode.
